# EUROPÄISCHE PATENTSCHRIFT

(11) **EP 3 107 508 B1**
(45) Veröffentlichungstag und Bekanntmachung des Hinweises auf die Patenterteilung: **04.04.2018**
(21) Anmeldenummer: 15703457.0
(22) Anmeldetag: 06.02.2015
(51) Int. Cl.: A61F 5/02

(54) **RUMPFORTHESE**
TRUNK ORTHOSIS
ORTHÈSE DE TRONC

(30) Priorität: 18.02.2014 DE 102014002177
(43) Veröffentlichungstag der Anmeldung: 28.12.2016
(73) Patentinhaber: Otto Bock HealthCare GmbH, 37115 Duderstadt (DE)
(72) Erfinder: VOLLBRECHT, Matthias, 37412 Herzberg (DE); LIDOLT, Klaus, 37115 Duderstadt (DE); VON ASCHEBERG, Alexander, 37115 Duderstadt (DE)
(74) Vertreter: Lins, Edgar
(86) Internationale Anmeldenummer: PCT/EP2015/000238
(87) Internationale Veröffentlichungsnummer: WO 2015/124265

(56) Entgegenhaltungen:
- EP-A1- 1 937 194
- US-A- 3 351 053
- US-A1- 2013 283 492

## Beschreibung

Die Erfindung betrifft eine Rumpforthese mit einer zum Anlegen an den Rumpf eines Patienten ausgebildeten Bandage und einer mit der Bandage verbundenen Stützeinrichtung. Eine solche Rumpforthese ist insbesondere bei Spinalkanalstenosen vorteilhaft und dient zum Umschließen eines Rumpfes eines Patienten.
Es ist bekannt, Rumpforthesen zur Stützung und Entlastung der Lendenwirbelsäule einzusetzen. Es kann notwendig sein, einen Lordosenbereich der Wirbelsäule vollständig zu entlasten, indem er durch eine Stützeinrichtung überbrückt wird, wodurch eine weitgehende Immobilisierung der Wirbelsäule in diesem Bereich bewirkt wird. Darüber hinaus ist es bekannt, den Lumbalbereich oder den Lumbosakralbereich der Wirbelsäule bei eingeschränkter Beweglichkeit zu unterstützen. Bei fortschreitender Genesung kann es gegebenenfalls erforderlich sein, eine gewisse Stützwirkung mittels einer Bandage oder einer geringfügig verstärkten Bandage auszuüben.

Die DE 202 04 747 U1 beschreibt eine Rumpforthese, die auf eine vielseitige Einsetzbarkeit für unterschiedliche Anwendungsfälle ausgerichtet ist. Die Bandage weist zwei überlappende Teilbandagen auf, die eine Anpassung der Bandagenhöhe an den jeweiligen Patienten ermöglicht. In Taschen können einschiebbare Stützstäbe positioniert werden, um unterschiedliche Stützeinrichtungen zur Überbrückung oder Stabilisierung des Lordosenbereiches zu befestigen. Ebenfalls kann eine Bauchpelotte vorgesehen sein. Die unterschiedlichen Stützeinrichtungen können über Klettbandverschlüsse an der Bandage befestigt und einfach ausgewechselt werden.
Die DE 10 2005 031 867 A1 betrifft eine Rumpforthese zum Umschließen eines Rumpfes eines Patienten mit einer Bandage und einer Stützeinrichtung, die durch eine Vielzahl von nebeneinander angeordneten, in Längsrichtung der Wirbelsäule ausgerichteten und direkt miteinander verbundenen Fingerstäben ausgebildet ist. Die Fingerstäbe bestehen aus einem flexiblen Kunststoff.
Die DE 10 2012 009 214 A1 beschreibt eine Spannvorrichtung für körperumgreifende Rückenorthesen. Die Orthese umfasst zumindest zwei Seitenteile, die jeweils mit einem im Bereich der Wirbelsäule des Patienten angeordneten Zentralteil über Flaschenzüge spannbar verbunden sind. Sowohl die Seitenteile als auch das Zentralteil können dabei aus mehreren übereinander angeordneten Elementen bestehen.

Die US 3,351,053 A, die als nächstliegender Stand der Technik angesehen wird, betrifft eine Rückenorthese, die im vorderen Bereich den Bauch des Patienten mittels Gurten und Riemen umschließt und im hinteren Bereich eine aus verschiedenen Bügeln und Stäben gebildete Konstruktion aufweist, wobei die Elemente teilweise gelenkig miteinander verbunden sind.
Die DE 20 2007 008 409 U1 betrifft ein Wirbelsäulenstützgerät, das einem Menschen, der eine kniende Tätigkeit ausübt, das Aufrichten erleichtern soll, indem ein mit Hilfe von Gummibändern vorgespannter Bügel eine aufrichtende Kraft im Brustbereich auf den Benutzer ausübt.
Die WO 93/06798 A2 betrifft ebenfalls eine Aufrichthilfe, die zwei im Hüftbereich des Benutzers gelagerte Bügel aufweisen, die eine federnde Rückstellkraft auf den Oberkörper des Benutzers ausüben.
Aufgabe der vorliegenden Erfindung ist es, eine Rumpforthese bereitzustellen, die eine dynamische Gelenkfunktion wahrnimmt und gleichzeitig im Lendenwirbelbereich eine Entlastung für den Patienten bereitstellt.
Erfindungsgemäß wird diese Aufgabe durch eine Rückenorthese mit den Merkmalen des Hauptanspruches gelöst. Vorteilhafte Ausgestaltungen und Weiterbildungen der Erfindung sind in den Unteransprüchen, der Beschreibung und den Figuren offenbart.
Die Rumpforthese mit einer zum Anlegen an den Rumpf eines Patienten ausgebildeten Bandage und einer mit der Bandage verbundenen Stützeinrichtung sieht vor, dass die Stützeinrichtung als Rahmen mit Rahmenabschnitten ausgebildet ist, die gelenkig oder elastisch aneinander gelagert und die Rahmenabschnitte federbelastet und im angelegten Zustand durch die Bandage vorgespannt sind. Durch den Rahmen ist es möglich, eine quasisteife Konstruktion bereitzustellen, über die es möglich ist, definiert Kräfte an den Anlagepunkten des Rahmens bzw. der Rahmenabschnitte an dem Patienten einzuleiten. Die gelenkige oder elastische Anordnung der Rahmenabschnitte zueinander lässt eine Beweglichkeit der Wirbelsäule zu, wobei gleichzeitig ein Stützeffekt erzielt wird. Dadurch ist es möglich, Wirbel, insbesondere Lendenwirbel oder untere Brustwirbel zu entlasten. Die Entlastung kann beispielsweise dadurch erfolgen, dass das Bandagenelement straff um den Rumpf herumgelegt wird, so dass eine Zugkraft auf den Rahmen bzw. die Rahmenabschnitte aufgebracht wird. Die Rahmenabschnitte werden dann durch die Zugkraft vorgespannt und bewirken, dass beispielsweise eine Flexionsunterstützung erfolgt und der Zwischenwirbelbereich der Lendenwirbelsäule teilweise aufgeweitet wird. Die Bandage wird vorteilhafterweise ventral geführt, also um den Bauch herum gelegt, während der Rahmen dorsal geführt ist. Die Rahmenabschnitte können eine doppelte C-Form aufweisen, wenn das Gelenk oder die elastische Biegestelle zwischen den Rahmenabschnitten ungefähr in einer vor der Wirbelsäule liegenden oder in der Höhe der Wirbelsäule liegenden Frontalebene angeordnet ist. Die Bandage ist nicht vollständig um den Rumpf herumgeführt, vielmehr reicht es aus, wenn lediglich im frontalen Bauchbereich die Bandage vorhanden ist, der Rahmen bildet den rückwärtigen Abschluss. Der Rahmen kann einteilig ausgebildet sein, so dass die Rahmenabschnitte zueinander elastisch verlagerbar sind. Die elastischen Rückstellkräfte werden von dem Rahmen bei einer Verformung aufgrund der Spannung des Bandage um dem Rumpf aufgebracht. Bei einer mehrteiligen Ausgestaltung des Rahmens können die Rahmenabschnitte als Rahmenelemente ausgebildet sein, die getrennt voneinander herstellbar und aneinander montierbar sind. Die Montage kann über Stecksysteme, andere formschlüssige Verbindungen reversibel oder auch dauerhaft über Verkleben, Verlöten oder Verschweißen erfolgen. Wenn die Rahmenabschnitte aus mehreren Rahmenelementen bestehen, besteht auch die Möglichkeit, die Rahmenelemente individuell an den Nutzer anzupassen. Die Rahmenelemente können verstellbar und aneinander fixierbar ausgebildet sein. Insbesondere besteht bei einer Teilung in der Sagittalebene die Möglichkeit, dass die Orthese in der Breite anpassbar an den jeweiligen Patienten ist, beispielsweise durch Schraubhülsen oder Steckhülsen oder Zwischenstücke, die zur Einstellung der Breite eingesetzt werden können. Die Rahmenelement selbst und auch die Verbindungselement können elastisch ausgebildet sein, um eine gewisse Anpassbarkeit und Flexibilität an den Nutzer und die Nutzungsbedingen zu ermöglichen.

Die Rahmenabschnitte oder Rahmenelemente sind federbelastet zueinander gelagert, wobei die Feder als ein Kraftbegrenzer dienen kann. Die Federcharakteristik kann linear, progressiv oder degressiv ausgestaltet sein Die Federkraft ist einstellbar, so dass das Maß der aufgebrachten Kraft auf den jeweiligen Patienten einstellbar ist. Vorteilhafterweise sind die Rahmenabschnitte oder Rahmenelemente dergestalt zueinander federbelastet, dass nach dem Anlegen der Rumpforthese eine Aufweitung im Lendenwirbelbereich stattfindet, insbesondere im unteren und mittleren Lendenwirbelbereich. Sofern der Rahmen einteilig ausgebildet ist, wird die Federwirkung zwischen dem oberen und unteren Rahmenabschnitt, also dem im unteren Brustbereich und dem Beckenbereich anliegenden Querabschnitt über einen elastischen Abschnitt oder elastische Abschnitt in den seitlichen Bereichen der Orthese bereit gestellt, beispielsweise durch eine Wicklung wie bei einer Schrauben- oder Spiralfeder. Hierzu ist vorteilhaft, wenn die Rahmenelement oder der Rahmen aus einem Vollmaterial hergestellt ist. Ist die Orthese mehrteilig aufgebaut, kann die Federkraft durch separate Federn oder Federelemente bereitgestellt werden, die an den Rahmenabschnitten befestigt sind. Separate Federelemente haben den Vorteil einer leichteren Herstellbarkeit und einer Auswechsel- und Anpassbarkeit, um für den jeweiligen Patienten die gewünschte Federkraft bereitstellen zu können.

Die Rahmenabschnitte oder Rahmenelemente können an Gelenkeinrichtungen gelagert sein, die medial und lateral zum Rumpf positioniert sind. Die Gelenkeinrichtungen können beispielsweise als separate Komponenten ausgebildet sein, in denen eine Feder integriert ist, deren Vorspannung einstellbar ist. An den Gelenkeinrichtungen können Aufnahmen für die Rahmenelemente oder Rahmenabschnitte vorgesehen sein, so dass eine Auswechselbarkeit an den Gelenkeinrichtungen zum Zwecke der Anpassung an die jeweiligen Patienten möglich ist. Alternativ dazu ist es vorgesehen, dass die Rahmenabschnitte oder Rahmenelemente einstellbar ausgebildet sind, beispielsweise in der Höhe und/oder der Breite, so dass die Rahmenkonstruktion einmalig aufgebaut und anschließend für alle Patienten einstellbar ist. Die Höhe der Rahmenelemente relativ zu den Gelenkeinrichtungen kann durch eine entsprechende Befestigung der Rahmenelemente an den Gelenkeinrichtungen erfolgen.

Vorteilhafterweise sind die Rahmenabschnitte oder Rahmenelemente als eine Rohrkonstruktion ausgebildet, wodurch eine besonders leichte Konstruktion bei hoher Stabilität erreicht wird. Dadurch ist es möglich, eine quasisteife Rahmenkonstruktion bereitzustellen, um die auf den Patienten aufzubringenden Kräfte einfach übertragen zu können. Sofern keine gesonderten Gelenkeinrichtungen mit einer starren Achse vorgesehen sind, kann die Gelenkeinrichtung auch durch eine verlagerbare Anordnung der Rahmenabschnitte oder Rahmenelemente zueinander an den Bandagenteilen oder an der Bandage erfolgen, wobei die Federkraft durch die Bandage selbst aufgebracht wird und die Bandage gleichzeitig dazu dient, die Rahmenelemente zueinander festzulegen.

An den Rahmenabschnitten können Polsterelemente angeordnet sein, um eine für den Patienten angenehme Abstützung der Rahmenelemente oder Rahmenabschnitte im Bereich des Kreuzbeines bzw. im Lumbosakralbereich sowie im oberen Lendenwirbelbereich oder im unteren Brustwirbelbereich zu ermöglichen.

Die Bandage kann mehrteilig ausgebildet sein und zumindest zwei Bandagenelemente aufweisen, die über Befestigungsmittel aneinander befestigbar sind. Die Befestigungsmittel dienen insbesondere zur formschlüssigen Festlegung der Bandagenelemente aneinander und können als Klettverschluss, Schnallen, Haken, Clipse, Knöpfe oder dergleichen ausgebildet sein.

Vorteilhafterweise ist die Bandage aus einem flexiblen Material ausgebildet, um das Anlegen der Rumpforthese zu erleichtern. Sofern das Material elastisch ist, kann die Bandage auch zu einer federnden und vorgespannten Lagerung an dem Patienten dienen.

Ein Spanngurt kann an der Stützeinrichtung befestigt sein und ist vorteilhafterweise auf oder in der Bandage geführt. Dadurch ist es möglich, nach dem eigentlichen Anlegen der Rumpforthese durch das Schließen der beiden Bandagenelemente vor dem Körper eine gewünschte Vorspannung aufzubringen. Der Spanngurt kann dazu als Flaschenzug ausgebildet sein und wird vorteilhafterweise auf der Bandage festgelegt, beispielsweise durch einen Klettverschluss, der an dem Ende des Spanngurtes angeordnet ist. Durch die mehrfach umgelenkte Führung des Spanngurtes auf der Bandage kann eine Kraftverstärkung erfolgen, ohne dass die Kraftaufbringung für den Nutzer anstrengend wäre. Der Spanngurt kann an dem Gelenk, der Gelenkeinrichtung oder im Bereich des Gelenkes der Rahmenelemente befestigt oder geführt sein. Die Zugkraft durch den Spanngurt wird vorteilhafterweise durch die Gelenkachse oder im Bereich der Gelenkachse aufgebracht. Ebenfalls ist es möglich, den Spanngurt direkt an der Gelenkeinrichtung zu befestigen oder dort durch eine Umlenkeinrichtung, beispielsweise einen Ring oder einen D-Ring hindurchzuführen. Andere Führungen an der Gelenkeinrichtung sind ebenfalls möglich, um eine definierte Krafteinleitung im Bereich der Gelenkachse vorzunehmen, wodurch eine exakte Krafteinleitung und dadurch Wirksamkeit der Rahmenelemente an den jeweiligen Auflagepunkten gewährleistet ist. Ist kein Scharnier zwischen den Rahmenabschnitten ausgebildet, wird durch eine Zuordnung des Spanngurtes zu dem Rahmen über Durchführungen oder Führungselemente erreicht, so dass die Krafteinleitung dauerhaft an der gewünschten Stelle erfolgt.

Die Federelemente für die Vorspannung der Rahmenabschnitte oder Rahmenelemente zueinander können als Schenkelfedern oder Stabfedern ausgebildet sein. Die Federn können mit Anschlägen versehen sein, die einstellbar sind, so dass einerseits eine Kraftbegrenzung und andererseits eine Bewegungsbegrenzung verwirklichbar sind. Dadurch können auch gewünschte Bewegungen oder Bewegungsumfänge eingeschränkt werden.

Nachfolgend werden Ausführungsbeispiele der Erfindung anhand der Figuren näher erläutert. Es zeigen:
- Figur 1 -: eine Frontalansicht einer ersten Ausführungsform;
- Figur 2 -: eine Seitenansicht der Figur 1;
- Figur 3 -: eine Rückansicht der Figur 1;
- Figur 4 -: eine Detailansicht;
- Figur 5 -: eine Frontalansicht einer Variante;
- Figur 6 -: eine Seitenansicht der Variante gemäß Figur 5; sowie
- Figur 7 -: eine Rückenansicht der Variante gemäß Figur 5.

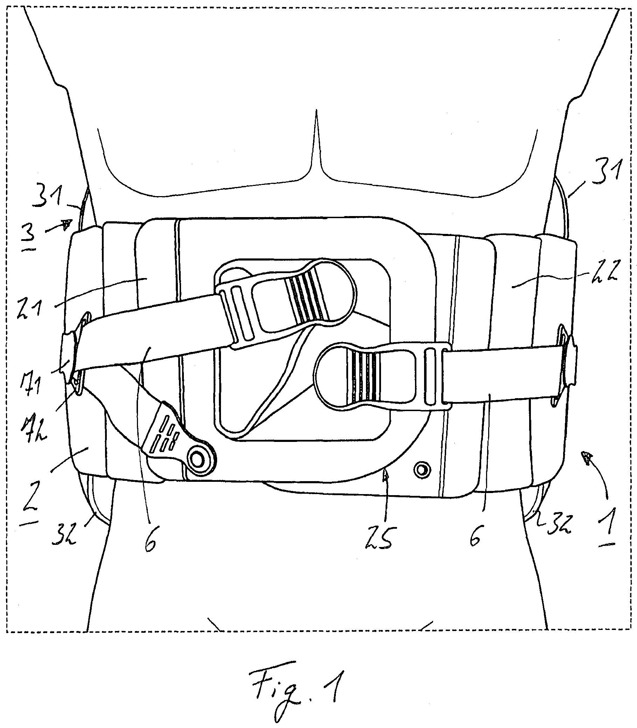

In der Figur 1 ist in einer Frontalansicht einer an einen Rumpf eines Patienten angelegte Orthese 1 gezeigt. Die Rumpforthese 1 sieht auf der Vorderseite eine Bandage 2 vor, die aus mehreren Bandagenelementen 21, 22 besteht. Die Bandagenelemente 21, 22 selbst können aus mehreren Komponenten bestehen, die lösbar oder fest aneinander angeordnet sind. Die Bandagenelemente 21, 22 sind links und rechts von dem Rumpf an einem Rahmen 3 angeordnet, der einen oberen Rahmenabschnitt 31 und einen unteren Rahmenabschnitt 32 aufweist. Zum Anlegen der Rumpforthese 1 werden die beiden Bandagenelemente 21, 22 geöffnet, der Rahmen 3 an den Rücken angelegt und anschließend werden die Bandagenelemente 21, 22 über Befestigungsmittel 25, die im dargestellten Ausführungsbeispiel als Klettverschlüsse ausgebildet sind, aneinander festgelegt. Dadurch umschlingt die Rumpforthese 1 den Körper des Patienten vollumfänglich, die Bandagenelemente 21, 22 sind jedoch nur im Abdomenbereich angeordnet. Nach dem Übereinanderlegen der beiden Bandagenelemente 21, 22 und einem formschlüssigen Festlegen über beispielsweise einen Klettverschluss 25, drückt der Rahmen 3 mit dem oberen Rahmenabschnitt 31 und dem unteren Rahmenabschnitt 32 an ihren Abstützpunkten gegen den Rücken bzw. gegen das Becken. Um diesen Druck zu verstärken, sind an der Vorderseite der Rumpforthese 1 Spanngurte 6 angeordnet, die auf dem oberen Bandagenabschnitt 21 festlegbar sind. Die Festlegung erfolgt nach dem Schließen der Bandagenelemente 21, 22 vorteilhafterweise über einen Klettverschluss, um eine nahezu stufenlose Anpassung der Anpresskraft über eine Einstellung der Länge bzw. Position der Spanngurte 6 zu erreichen. Der in der Ansicht der Figur 1 linke Spanngurt 6 ist mit seinem ersten Ende am unteren Rand des Bandagenelementes 21 festgelegt, wird dann durch eine Öse 72, die an einer Lasche 71 an dem 3 befestigt ist, hindurchgeführt und dann zurück auf das Bandagenelement 21 geführt und dort auf einem Flauschbereich mittels eines Hakenbereiches eines Klettverschlusses festgelegt. Durch diese Umlenkung kann eine erhöhte Zugkraft bei gleichzeitig präziser Einleitung der nach vorne ziehenden Kraft durch den Spanngurt 6 bewirkt werden. Der in der Ansicht rechte Spanngurt 6 ist einfach an einer korrespondierend ausgebildeten Lasche an dem Rahmen 3 festgelegt und wird auf der Vorderseite des oberen Bandagenelementes 21 über einen Klettverschluss fixiert.

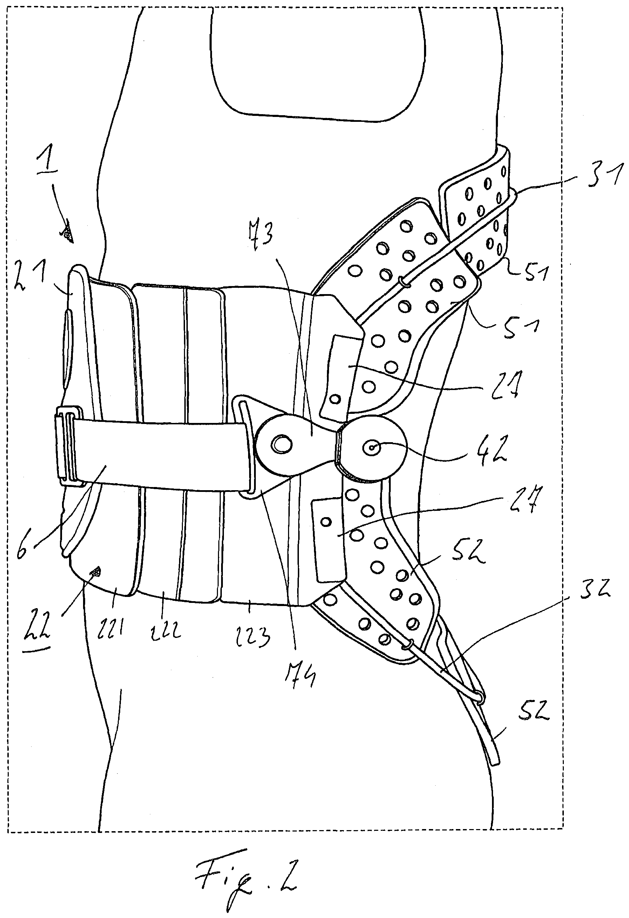

Figur 2 zeigt eine Seitenansicht der Rumpforthese 1 gemäß dem Ausführungsbeispiel der Figur 1. Es ist zu erkennen, dass das Bandagenelement 22 aus drei Einzelkomponenten 221, 222, 223 besteht, die beispielsweise über eine Vielzahl von Klettverschlüssen an einer festlegbar sind. Dadurch ist es möglich, eine modulare Ausgestaltung der Bandage 2 zu erreichen, um Längenanpassungen vorzunehmen. Das Bandagenelement 22 ist mit seinem hinteren Teil 223 an den beiden als separate Rahmenelemente ausgebildete Rahmenabschnitte 31, 32 über Laschen 27 befestigt. Die Rahmenelemente 31, 32 sind als Rohrkonstruktion mit einem runden Rohrquerschnitt, insbesondere mit einem Hohlquerschnitt, ausgebildet, um eine leichte und gleichzeitig stabile sowie gegebenenfalls elastisch federnde Ausgestaltung zu erreichen. Über die Laschen 27 wird zur Positionierung der Orthese an dem Körper eine erste Zugkraft über die Rahmenelemente 31, 32 in Frontalrichtung und damit auch ein geringer Druck auf den Rücken und das Becken des Patienten aufgebracht.

An den Rahmenelementen 31, 32 sind Polsterelemente 51, 52 oder auch Pelotten angeordnet, die eine Kraftverteilung und eine Komforterhöhung der Anlage der Rahmenelemente 31, 32 bewirken. Die Polsterelemente 51, 52 polstern sowohl im Brustwirbelbereich als auch im Lendenwirbelbereich sowie seitlich am Rumpf. Die Rahmenabschnitte 31, 32 verlaufen in einer doppelten C-Form und erstrecken sich von der Verbindungsstelle der beiden Rahmenabschnitte 31, 32 zunächst gradlinig nach oben bzw. unten und verlaufen in einem doppelten Bogen, der sowohl von oben nach unten bzw. unten nach oben und von vorne nach hinten verläuft, zur Verbindungsstelle auf der anderen Seite.

Die beiden Rahmenabschnitte 31, 32 sind an einer Gelenkeinrichtung 42 verschwenkbar aneinander gelagert. Eine korrespondierende Gelenkeinrichtung ist auf der andere Seite ebenfalls vorgesehen und ermöglicht eine im Wesentlichen freie Verschwenkbarkeit der Rahmenelemente 31, 32 um die Gelenkeinrichtung 42. Über eine Lasche 73 und eine gelenkig daran gelagerte Öse 74 wird der Spanngurt 6 frontal gespannt und eine zentral auf die Schwenkachse der Gelenkeinrichtung 42 wirkende Kraft ermöglicht eine präzise Krafteinleitung in den Gelenkbereich. Der größere Anteil der wirksamen Kraft, die auf den Rücken und das Becken ausgeübt wird, wird über den Spanngurt 6 und die Laschen 73, die lateral an dem Körper geführt sind, ausgeübt. Der Spanngurt 6 wirkt über die Lasche 73 direkt auf den Gelenkbereich und zieht den im wesentlichen in der Mitte zwischen dem oberen und unteren Querbügel Gelenkbereich nach vorne.

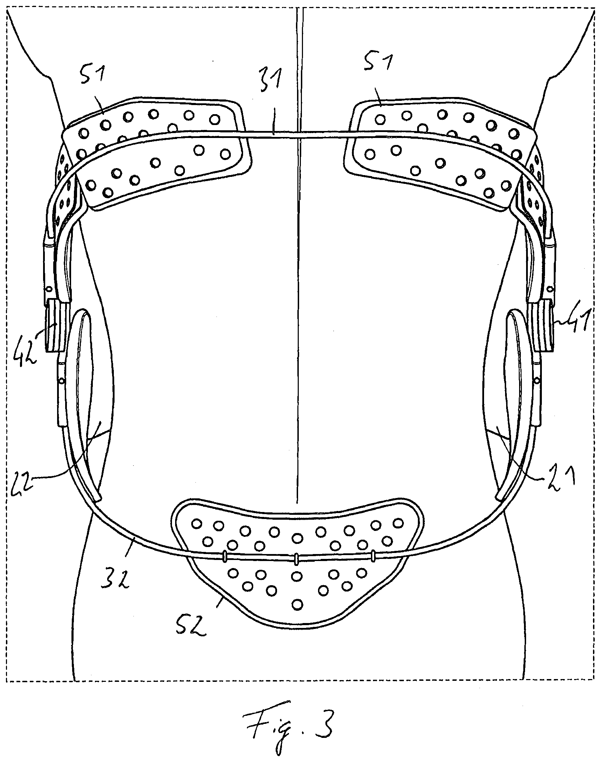

Figur 3 zeigt die Orthese 1 in einer Rückansicht, in der die beiden separat gefertigten Rahmenelemente 31, 32 ebenso wie die im Brustwirbelbereich oberen Polsterelemente 51 und das im Lendenwirbelbereich untere Polsterelement 52 und die seitlichen Polsterelemente zu erkennen sind, statt oder neben der Polsterelemente können auch Pelotten an dem Rahmen oder den Rahmenelementen befestigt sein. Die Rahmenelemente 31, 32 sind beidseitig über Gelenkeinrichtungen 41, 42 verschwenkbar aneinander gelagert und über die nicht dargestellten Spanngurte 6 und Bandagenelemente 21, 22 um den Körper des Patienten herum angelegt. Die Orthese 1 ist somit umfänglich um den Rumpf des Patienten geschlossen ausgebildet. Die Gelenkeinrichtungen 41, 42 können mit Federn versehen sein, beispielsweise Druckfedern, Zugfedern, Spiralfedern oder Elastomerelemente, um gegeneinander vorgespannt sein zu können. Die Federkraft wirkt vorteilhafterweise so, dass ein nach vorne Beugen unterstützt wird, also dass im Brustwirbelbereich und Lendenwirbelbereich eine nach vorne wirkende Kraft anliegt. Alternativ zu einem separaten Federelement ist es möglich, die Rahmenelemente 31, 32 so miteinander zu verbinden, dass eine elastische Vorspannkraft herrscht. Ebenfalls ist es möglich, den Rahmen 3 einteilig auszubilden und durch eine entsprechende Formgestaltung der Rahmenabschnitte 31, 32 den gewünschten Effekt einer Flexion in Frontalrichtung zu erreichen.

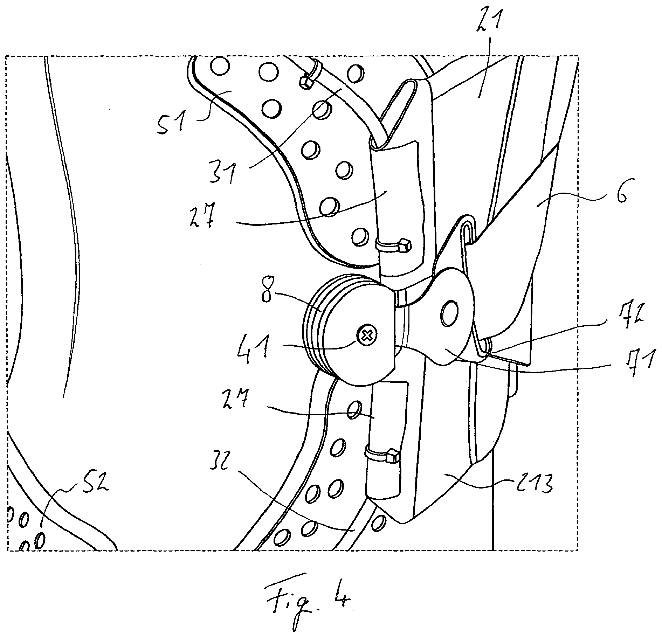

Figur 4 zeigt eine Detailansicht der Orthese im Bereich der Verbindungsstelle der beiden Rahmenelemente 31, 32. Die Laschen 27 des hinteren Bandagenelementes 213 sind an den Rahmenelementen 31, 32 befestigt, ebenso die Lasche 71 im Bereich des Drehpunktes der Gelenkeinrichtung 41. Eine Öse 72 ist schwenkbar an der Lasche 71 befestigt und dient zur Umleitung des Spanngurtes 6. Innerhalb der Gelenkeinrichtung 41 ist eine Drehfeder 8 angeordnet, um die beiden Rahmenelemente 31, 32 in der gewünschten Richtung zu belasten, im dargestellten Ausführungsbeispiel zur Belastung in Frontalrichtung, um eine Beugung und Aufweitung des Spinalkanals zu erreichen. Die Laschen 27 können über reversible Verschlusselemente wie Klettverschlüsse, Druckknöpfe oder dergleichen an den Rahmenelementen 31, 32 festgelegt sein, grundsätzlich ist es auch möglich, eine dauerhafte Verbindung durch Verschweißen, Vernähen oder beispielsweise Kabelbinder zu realisieren.

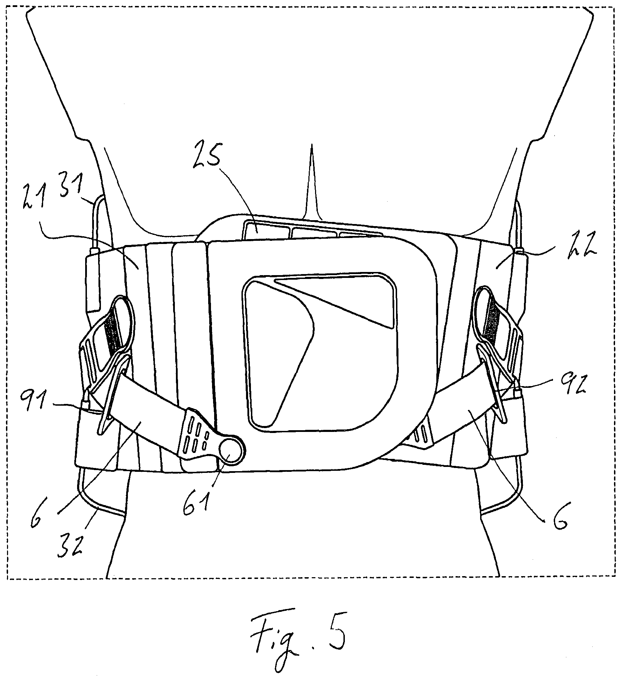

Figur 5 zeigt eine Variante der Erfindung, bei der die Befestigungsmittel 25 in Gestalt von Klettverschlüssen dargestellt sind, ebenso ist zu erkennen, dass beide Spanngurte 6 über Umlenkeinrichtungen, wie sie in der Figur 4 im Detail dargestellt sind, an den jeweiligen Verbindungen oder im der Mitte zwischen den Rahmenabschnitten 31, 32 gelagert sind, um eine flaschenzugartige Konstruktion zu erreichen. Dadurch ist es möglich, eine Kraftverstärkung zu erreichen, wenn die Spanngurte 6 gezogen und frontal auf Flauschbereichen an der Oberseite des Bandagenelements 21 festgelegt werden.

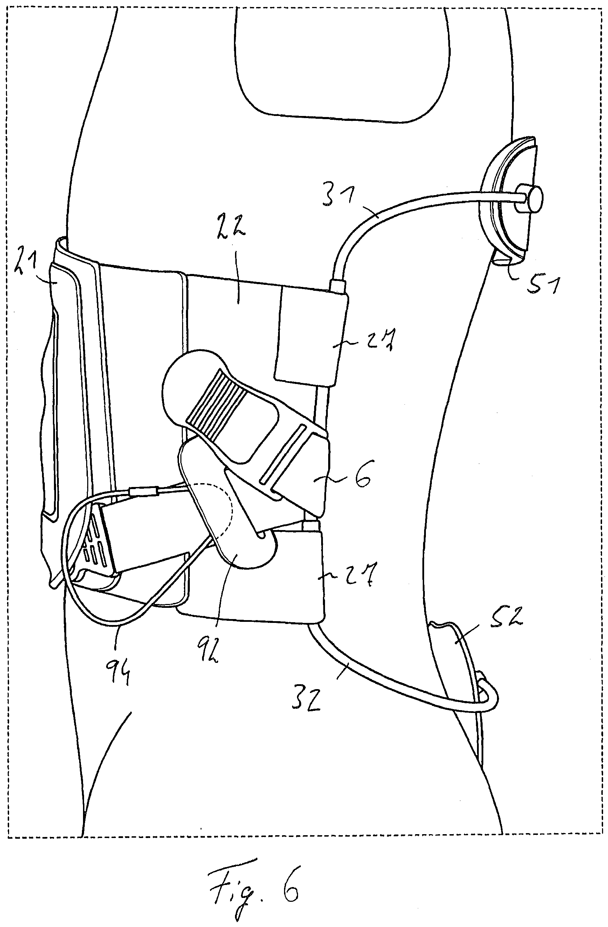

An den Spanngurten 6 sind Schieber 91, 92 angeordnet, die im Zusammenhang mit der Figur 6 näher erläutert werden.

Figur 6 zeigt eine Seitenansicht der Orthese gemäß Figur 5, in der zu erkennen ist, dass die Rahmenabschnitte 31, 32 ohne eine scharnierartige Gelenkeinrichtung elastisch aneinander gelagert sind, entweder über ein Stecksystem, beispielsweise eine elastische Verbindungsstange, oder durch an den Rahmenabschnitten 31, 32 befestigte oder ausgebildete Muffen. Das Bandagenelement 22 ist über die Laschen 27 an den Rahmenabschnitten 31, 32 befestigt, zwischen den Laschen 27 ist ein Freiraum ausgebildet, durch den der Spanngurt 6 hindurchgeführt wird, so dass eine örtliche Zuordnung der Umlenkstelle des Spanngurtes 6 gewährleistet ist. An der Umlenkstelle des Spanngurtes 6 der hinter dem Rahmen 3 hindurchgeführt und dann vorn auf der Vorderseite der Bandagenelemente 21, 22 über beispielsweise Klettverschlüsse festgelegt ist, findet eine zentrale Krafteinleitung statt. Der Schieber 92 ist mit einer Schlaufe 94 versehen, über die es möglich ist, einen verbesserten Griff zu realisieren, um Spannkräfte auf den Spanngurt 6 auszuüben.

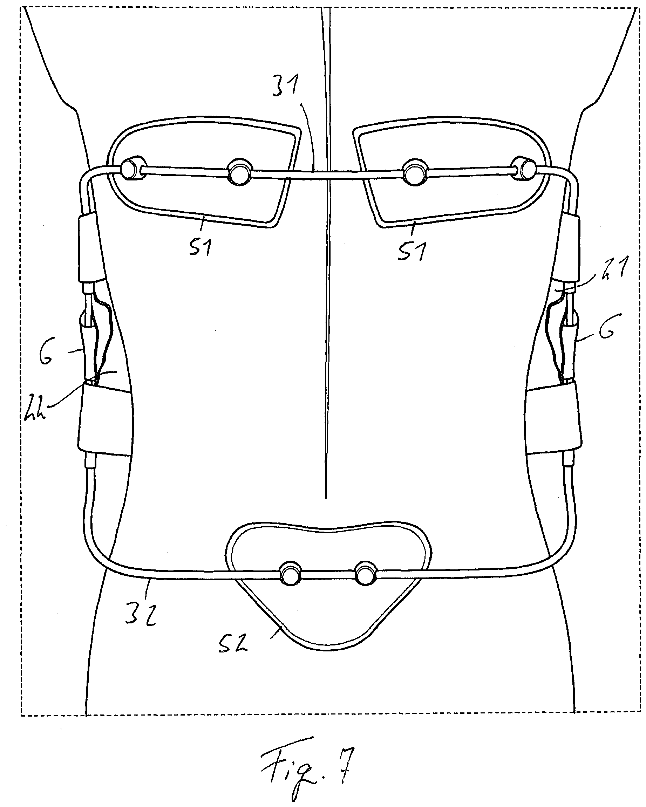

Figur 7 zeigt die Rückseite der Orthese mit zwei oberen Polsterelementen 51, die beidseitig der Wirbelsäule angeordnet sind, sowie einem unteren Polsterelement 52, das im Bereich des Kreuzbeins an dem Patienten anliegt. Es ist zu erkennen, dass die Spanngurte 6 um den Rahmen 3 im Bereich zwischen den oberen und unteren Rahmenabschnitten 31, 32 herumgelegt ist.

## Patentansprüche

1. Rumpforthese mit einer zum Anlegen an den Rumpf eines Patienten ausgebildeten Bandage (2) und einer mit der Bandage (2) verbundenen Stützeinrichtung (3), wobei die Stützeinrichtung (3) als Rahmen mit Rahmenabschnitten (31, 32) ausgebildet ist, die gelenkig oder elastisch aneinander gelagert sind, **dadurch gekennzeichnet, dass** die Rahmenabschnitte (31, 32) federbelastet und im angelegten Zustand durch die Bandage (2) vorgespannt sind.

2. Rumpforthese nach Anspruch 1, **dadurch gekennzeichnet, dass** die Rahmenabschnitte (31, 32) an Gelenkeinrichtungen (41, 42) gelagert sind, die medial und lateral zum Rumpf positioniert sind.

3. Rumpforthese nach Anspruch 1 oder 2, **dadurch gekennzeichnet, dass** an den Rahmenabschnitten (31, 32) Polsterelemente (51, 52) oder Pelotten angeordnet sind.

4. Rumpforthese nach einem der voranstehenden Ansprüche, **dadurch gekennzeichnet, dass** die Bandage (2) mehrteilig ausgebildet ist und zumindest zwei Bandagenelemente (21, 22) aufweist, die über Befestigungsmittel (25) aneinander befestigbar sind.

5. Rumpforthese nach Anspruch 4, **dadurch gekennzeichnet, dass** die Befestigungsmittel (25) als Formschlusselemente ausgebildet sind.

6. Rumpforthese nach einem der voranstehenden Ansprüche, **dadurch gekennzeichnet, dass** die Bandage (2) aus einem flexiblen und/oder elastischen Material ausgebildet ist.

7. Rumpforthese nach einem der voranstehenden Ansprüche, **dadurch gekennzeichnet, dass** zumindest ein Spanngurt (6) an der Stützeinrichtung (3) befestigt und auf der Bandage (2) geführt ist.

8. Rumpforthese nach Anspruch 7, **dadurch gekennzeichnet, dass** der Spanngurt (6) an dem Gelenk der Rahmenabschnitte (31, 32) befestigt oder geführt ist.

9. Rumpforthese nach Anspruch 7 oder 8, **dadurch gekennzeichnet, dass** der Spanngurt (6) als Flaschenzug ausgebildet ist.

10. Rumpforthese nach einem der Ansprüche 7 bis 9, **dadurch gekennzeichnet, dass** der Spanngurt (6) auf der Bandage (2) reversibel festlegbar ist.

11. Rumpforthese nach einem der voranstehenden Ansprüche, **dadurch gekennzeichnet, dass** die Stützeinrichtung (3) mehrteilig ausgebildet ist.

## Claims

1. Trunk orthosis with a bandage (2) designed to be applied to a patient's trunk, and with a support device (3) connected to the bandage (2), said support device (3) being designed as a frame with frame portions (31, 32) which are mounted on each other in an articulated manner or elastically, **characterized in that** the frame portions (31, 32) are spring-loaded and, in the applied state, are pre-stressed by the bandage (2).

2. Trunk orthosis as claimed in claim 1, **characterized in that** the frame portions (31, 32) are mounted on hinge mechanisms (41, 42) which are positioned medially and laterally with respect to the trunk.

3. Trunk orthosis as claimed in claim 1 or 2, **characterized in that** cushioning elements (51, 52) or pads are arranged on the frame portions (31, 32).

4. Trunk orthosis as claimed in one of the preceding claims, **characterized in that** the bandage (2) has a multi-part design and has at least two bandage elements (21, 22), which can be secured to each other via securing means (25).

5. Trunk orthosis as claimed in claim 4, **characterized in that** the securing means (25) are designed as form-fit elements.

6. Trunk orthosis as claimed in one of the preceding claims, **characterized in that** the bandage (2) is made from a flexible and/or elastic material.

7. Trunk orthosis as claimed in one of the preceding claims, **characterized in that** at least one tightening strap (6) is secured on the support device (3) and is guided on the bandage (2).

8. Trunk orthosis as claimed in claim 7, **characterized in that** the tightening strap (6) is secured or guided on the hinge of the frame portions (31, 32).

9. Trunk orthosis as claimed in claim 7 or 8, **characterized in that** the tightening strap (6) is designed as a pulley system.

10. Trunk orthosis as claimed in one of claims 7 to 9, **characterized in that** the tightening strap (6) can be secured reversibly on the bandage (2).

11. Trunk orthosis as claimed in one of the preceding claims, **characterized in that** the support device (3) has a multi-part design.

## Revendications

1. Orthèse de tronc comportant un bandage (2) réalisé pour l'appliquer contre le tronc d'un patient et un moyen de soutien (3) relié au bandage (2), le moyen de soutien (3) étant réalisé sous forme de cadre ayant des portions de cadre (31, 32) qui sont montées de façon articulée ou élastique les unes contre les autres,
**caractérisée en ce que**
les portions de cadre (31, 32) sont sollicitées élastiquement ou sont précontraintes par le bandage (2), dans l'état appliqué.

2. Orthèse de tronc selon la revendication 1,
**caractérisée en ce que**
les portions de cadre (31, 32) sont montées sur des moyens formant articulation (41, 42) qui sont mis en position médiale et latérale par rapport au tronc.

3. Orthèse de tronc selon la revendication 1 ou 2,
**caractérisée en ce que**
des éléments de rembourrage (51, 52) ou des coussinets sont agencés sur les portions de cadre (31, 32).

4. Orthèse de tronc selon l'une des revendications précédentes,
**caractérisée en ce que**
le bandage (2) est réalisé en plusieurs parties et comprend au moins deux éléments de bandage (21, 22) qui sont fixables l'un contre l'autre par des moyens de fixation (25).

5. Orthèse de tronc selon la revendication 4,
**caractérisée en ce que**
les moyens de fixation (25) sont réalisés sous forme d'éléments en coopération de formes.

6. Orthèse de tronc selon l'une des revendications précédentes,
**caractérisée en ce que**
le bandage (2) est réalisé en un matériau flexible et/ou élastique.

7. Orthèse de tronc selon l'une des revendications précédentes,
**caractérisée en ce que**
au moins une sangle de serrage (6) est fixée sur le moyen de soutien (3) et guidée sur le bandage (2).

8. Orthèse de tronc selon la revendication 7,
**caractérisée en ce que**
la sangle de serrage (6) est fixée ou guidée sur l'articulation des portions de cadre (31, 32).

9. Orthèse de tronc selon la revendication 7 ou 8,
**caractérisée en ce que**
la sangle de serrage (6) est réalisée sous forme de palan.

10. Orthèse de tronc selon l'une des revendications 7 à 9,
**caractérisée en ce que**
la sangle de serrage (6) est immobilisable de façon réversible sur le bandage (2).

11. Orthèse de tronc selon l'une des revendications précédentes,
**caractérisée en ce que**
le moyen de soutien (3) est réalisé en plusieurs parties.
